# EUROPEAN PATENT SPECIFICATION

(11) **EP 1 087 773 B1**
(45) Date of publication and mention of the grant of the patent: **12.01.2005**
(21) Application number: 99928256.9
(22) Date of filing: 14.06.1999
(51) Int. Cl.: A61K 31/565, A61K 9/00, A61K 47/40, A61P 5/30

(54) **VAGINAL ACTIVE AGENT DELIVERY PROCEDURES AND FORMULATIONS THEREOF**
ABGABEVERFAHREN UND FORMULIERUNGEN FÜR VAGINALWIRKSTOFFE
OPERATIONS D'APPORT INTRA-VAGINAL ET PREPARATIONS DESTINEES A CET EFFET

(30) Priority: 18.06.1998 NZ 33072698
(43) Date of publication of application: 04.04.2001
(73) Proprietor: Interag, Hamilton (NZ)
(72) Inventor: BUNT, Craig, Robert, Hamilton (NZ); RATHBONE, Michael, John, Hamilton (NZ); BURGGRAAF, Shane, Hamilton (NZ)
(74) Representative: McLeish, Nicholas Alistair Maxwell
(86) International application number: PCT/NZ1999/000083
(87) International publication number: WO 1999/065497

(56) References cited:
- EP-A1- 0 349 091
- WO-A-95/31178
- WO-A-99/63967
- AU-A- 7 411 194
- DE-C1- 19 734 538
- US-A- 3 892 855
- US-A- 4 642 305
- US-A- 4 877 774
- US-A- 5 472 954
- PHARMAZIE, Vol. 51, Number 1, 1996, FRIDRIKSDOTTIR et al., "Design and In Vivo Testing of 17beta-Estradiol-HPBCD Sublingual Tablets", pages 39-42. XP000545921
- EUROPEAN JOURNAL OF PHARMACEUTICS AND BIOPHARMACEUTICS, Vol. 42(5), 1996, KUBLIK et al., "Nasal Absorption of 17beta-Estradiol from Different Cyclodextrin Inclusion Formulations in Sheep", pages 320-324. XP000632691
- DATABASE CAPLUS [Online] 1997 BREWSTER M.E. ET AL.: 'Intravenous and Buccal 2-Hydroxypropyl-beta-Cyclodextrin Formulations of E2 - CDS - Phase I Clinical Trails', XP002966204 Database accession no. 1997:194543 & PROC. OF THE INT. SYMPOSIUM ON CYCLODEXTRINNS 8TH 31 March 1996 - 02 April 1996, BUDAPEST, pages 507 - 510

## Description

### TECHNICAL BACKGROUND

The present invention relates to intra-vaginal delivery or dosage units, compositions suitable therefor, the use thereof and related means and methods.

In our New Zealand patent specification numbers 207341, 286492 (PCT/NZ97/00052) and 314572/314175 (PCT/NZ98/00011) there are disclosed procedures applicable in a range of different animals insofar as the synchronisation of the onset of oestrus is concerned.

In our EAZI-BREED™, CIDR™ "Controlled Breeding and Reproductive Management" publication there is disclosed the use of an oestradiol co-treatment (ie; CIDIROLT™) with the use of our progesterone releasing CIDR™ intra-vaginal inserts in cattle for treatment of anoestrus or for oestrus synchrony. Current practice when selecting an oestradiol co-treatment, for use with a progesterone releasing intra-vaginal insert (such as our CIDR-B™ device), is to select oestradiol benzoate by intramuscular (i.m.) injection (eg; CIDIROL™).

In the past attempts at vaginal administration of oestradiol benzoate by using a capsule has resulted in poor oestrus expression and poor fertility. This outcome has been notwithstanding doses typically 10 times higher than those usually administered by intra-muscular injection.

### BACKGROUND ART

Our invention is directed towards a means whereby delivery of an active agent such as that to serve the role of previously used oestradiol benzoate is improved using and/or despite using a vaginal administration procedure.

It is therefore an object of the present invention to provide intra-vaginal delivery systems, dosage units, compositions, methods of use thereof and related means and methods which will be useful (preferably in conjunction with a progesterone releasing intra-vaginal insert such as our CIDR-B™ range of intra-vaginal inserts) in animals.

WO 95/31178 discloses the use of cyclodextrin as a mucoadhesive.

US 5,472,954 discloses a method for enhancing the complexation of cyclodextrin with a lipophilic and/or water active ingredient.

US 4,877,774 discloses formulations containing crystalline complexes of γ-cyclodextrin and/or hydroxypropyl-β-cyclodextrin and an active material.

Pharmazie 51(5): 39-42 (1996) discloses formulations containing complexes of hydroxypropyl-β-cyclodextrin and oestradiol 17β.

US 3,892,855 discloses a method of fertile breeding control by firstly administration of a progestin and then intramuscular injection of a luteolytic agent.

WO99/63967 discloses a device combining cyclodextrin and progesterone, as active ingredient.

We have determined in cattle that the lack of performance when using vaginal administration of oestradiol benzoate is not attributed to the dose, as typically doses are 10 times higher than those administered by intra-muscular injection, but to poor and variable absorption of oestradiol benzoate following vaginal administration. Typically peak plasma oestradiol concentrations following vaginal administration of 10 mg oestradiol benzoate range from 2 to 5 pg/ml. The peak in plasma levels is obtained between 2 and 48 hours following administration. In comparison peak plasma oestradiol concentrations following i.m. injection of 1 mg oestradiol benzoate range from 8 to 13 pg/ml. The peak in plasma level is obtained by approximately 2 hours following administration and maintained for up to 24 hours following treatment.

We believe similar effects occur in other animal species, viz, buffalo, pig, goat, sheep and deer.

As a result of our research in cattle we have determined that the natural oestradiol 17β rather than the synthetic analogue oestradiol benzoate can be effectively administered vaginally despite the fact that if given intra-muscularly oestradiol 17β was known to be shorter acting than the intra-muscularly efficacious oestradiol benzoate analogue thereof.

Our research in cattle has established that irrespective of whether or not that it is oestradiol 17β or an analogue thereof (such as oestradiol benzoate) that is delivered intravaginally an efficacious delivery thereof is possible in conjunction with an appropriate agent.

Our research has also established in cattle that intra-vaginal delivery of oestradiol 17β in preference to its analogues or the delivery of oestradiol 17β and/or its analogues in conjunction with at least one cyclodextrin can maintain serum levels of the active metabolite above normal for at least 24 hours.

We believe each, any or all of these findings are also appropriate for other animal species, viz, buffalo, pig, goat, sheep and deer, where such a regime might from time to time be warranted.

### DISCLOSURE OF THE INVENTION

Accordingly in a first aspect the present invention consists in **an intra-vaginal composition** for intra vaginal administration into a mammal, said composition being or having, as a solids admixture, a active principle (eg; a steroid such as oestradiol benzoate, oestradiol 17β or a prodrug of either) in admixture with γ-cyclodextrin and/or hydroxypropyl β-cyclodextrin.

Preferably said composition is in the form of a tablet or as part of a capsule.

Preferably said active principle is oestradiol 17β.

Preferably said active principle is to achieve, in a target mammal, an efficacious effect insofar as oestrus expression is concerned or an efficacious effect insofar as oestrus synchronisation is concerned.

Preferably said composition is provided as a dosage unit for a target mammal where the active principle is oestradiol 17β in an amount of between 0.72 and 7.2 mg.

Preferably the active principle is oestradiol 17β and there is between 0.5 to 1.5 moles of γ-cyclodextrin and/or hydroxypropyl β-cyclodextrin per mole of oestradiol 17β.

In another aspect the invention consists in **the use of a composition** of the present invention wherein, after an insertion of said composition as an intra-vaginal dosage unit in a target mammal, the plasma oestradiol concentration in the mammal 2 hours following the intra-vaginal administration is greater than 5 pg/ml and 24 hours following the intra-vaginal administration is less than 5 pg/ml.

In yet another aspect the invention consists in, in a mammalian herd oestrus synchrony procedure which involves the insertion into and the subsequent withdrawal from each mammal of the herd of an intra vaginal device adapted to deliver progesterone, the use of an oestradiol co-treatment which involves a timely intra vaginal insertion into each such mammal of an intra vaginal dosage form having an active principal selected from the group consisting of (i) oestradiol benzoate, (ii) oestradiol 17β, (iii) prodrugs of (i) and, (iv) prodrugs of (ii) in admixture with a cyclodextrin selected from the group consisting of γ-cyclodextrin and hydroxypropyl β-cyclodextrin.

In yet another aspect the invention consists in, in a mammalian oestrus expression procedure which involves the insertion into and the subsequent withdrawal from each mammal of the herd of an intra vaginal device adapted to deliver progesterone, the use of an oestradiol co-treatment which involves a timely intra vaginal insertion into each such mammal of an intra vaginal dosage form having an active principal selected from the group consisting of (i) oestradiol benzoate, (ii) oestradiol 17β, (iii) prodrugs of (i) and, (iv) prodrugs of (ii) in admixture with a cyclodextrin selected from the group consisting of γ-cyclodextrin and hydroxypropyl β-cyclodextrin.

Preferably the active principal is oestradiol 17β.

Preferably there are between 0.5 to 1.5 moles of γ-cyclodextrin and/or hydroxypropyl β-cyclodextrin per mole of oestradiol 17β.

Preferably the oestradiol co-treatment is also a use.

Preferably said oestradiol co-treatment intra vaginal dosage form is a tablet or capsule.

Preferably each such tablet or capsule does not require active removal prior to, during or subsequent to the removal of said intra vaginal device adapted to deliver progesterone.

In a further aspect the invention consists in the use of γ-cyclodextrin and/or hydroxypropyl β-cyclodextrin as absorption enhancers in the preparation of a pharmaceutical composition for the intra-vaginal administration of at least one active principle, said composition being a composition as aforesaid.

Preferably said formula dosage unit is in the form of a capsule, tablet or similar product and may, for example, be associated with a delayed release mechanism of some intra-vaginal device adapted to release some preceding medicament.

In another aspect the invention is a tablet or capsule (preferably capable in a target species recipient mammal of providing a plasma oestradiol concentration in the mammal two hours following intra vaginal administration of the table or capsule that is greater than 5 pg/ml and which 24 hours following the intra vaginal administration of the tablet or capsule will be less than 5 pg/ml) said tablet or capsule being formed of or having from 0.72 to 7.2 mg of oestradiol 17β in admixture with a cyclodextrin selected from one or both of γ-cyclodextrin and/or hydroxypropyl β-cyclodextrin, the mole ratio of the cyclodextrin(s) to oestradiol ranging from 0.5:1 to 1.5:1.

In another aspect the invention is an intra vaginal table or capsule formed or having an analogue of oestradiol 17β in an amount equivalent to 0.72 to 7.2 mg of oestradiol 17β and from 6 to 150 mg cyclodextrin(s).

Preferably the ratio of agent (eg; cyclodextrin) to enhance absorption to active material or pro-drug is less than 3:2 (agent:active) by molecular amount, that is 3 moles of cyclodextrin to every 2 moles of active.

Preferably the intra-vaginal dosage unit has from 1.2 to 7.2 mg of oestradiol 17β [or an analogue equivalent amount, eg; from 10 to 30 mg if analogue is oestradiol benzoate] and from 6 to 150 mg cyclodextrin(s),
and optionally other excipients, solid or liquid,
and, preferably, if a capsule, is encased in a material such as gelatin that will release the capsule contents into vaginal fluids.

A suitable source of cyclodextrins are the products BETA W7 HP hydroxypropyl β-cyclodextrin, BETA W7 β-cyclodextrin and GAMMA W8 γ-cyclodextrin from Wacker Chemicals Australia, Victoria, Australia.

A suitable source of oestradiol benzoate is from ICN Biomedical. Ohio, USA.

A suitable source of oestradiol 17β is from Sigma Chemical Company, USA.

The invention consists in the foregoing and also envisages constructions of which the following gives examples.

### DETAILED DESCRIPTION OF THE INVENTION

Preferred forms of the present invention will now be described with reference to the accompanying drawings in which:
**Figure 1** is a plasma oestradiol concentrations following intramuscular injection of 0.72 mg (closed square) or vaginal administration of 7.2 mg (open square) of oestradiol 17β. Error bars are standard error means (n=3).
**Figure 2** is a plasma oestradiol concentrations following vaginal administration of 10 mg oestradiol benzoate (open square), 10 mg oestradiol benzoate with 1:1 molar ratio β-cyclodextrin (open diamond), 10 mg oestradiol benzoate with 1:1 molar ratio hydroxypropyl β-cyclodextrin (open circle) or 10 mg oestradiol benzoate with 1:1 molar ratio γ-cyclodextrin (open triangle). Error bars are standard error means (n=4).
**Figure 3** is a plasma oestradiol concentrations following vaginal administration of oestradiol 17β 7.2 mg (open square), 7.2 mg with 1:1 molar ratio β-cyclodextrin (open diamond), 7.2 mg with 1:1 molar ratio hydroxypropyl β-cyclodextrin (open circle) or 7.2 mg with 1:1 molar ratio γ-cyclodextrin (open triangle). Error bars are standard error means (n=4).
Figure 4 is a plasma oestradiol concentration following vaginal administration of 1.2 mg (closed diamond), 2.5 mg (closed square) or 7.2 mg (closed triangle) oestradiol 17β with 0.5:1 molar ratio of γ-cyclodextrin to oestradiol 17β. Error bars are standard error means (n=4).
**Figure 5** is a plasma oestradiol concentration following vaginal administration of 1.2 mg (closed diamond), 2.5 mg (closed square) or 7.2 mg (closed triangle) oestradiol 17β with 1:1 molar ratio of γ-cyclodextrin to oestradiol 17β. Error bars are standard error means (n=4).
**Figure 6** is a plasma oestradiol concentration following vaginal administration of 1.2 mg (closed diamond), 2.5 mg (closed square) or 7.2 mg (closed triangle) oestradiol 17β with 3:2 molar ratio of γ-cyclodextrin to oestradiol 17β. Error bars are standard error means (n=4).
**Figure 7** is a area under the plasma oestradiol concentration against time curve (AUC) following vaginal administration of 1.2 mg, 2.5 mg or 7.2 mg oestradiol 17β with γ-cyclodextrin to oestradiol 17β molar ratio of 0.5 (closed diamond), 1 (closed square) or 1.5 (closed triangle). Error bars are standard error means (n=4).
**Figure 8** is a time to maximum plasma concentration (tmax) following vaginal administration of 1.2 mg, 2.5 mg or 7.2 mg oestradiol 17β with γ-cyclodextrin to oestradiol 17β molar ratio of 0.5 (closed diamond), 1 (closed square) or 1.5 (closed triangle). Error bars are standard error means (n=4).
Figure 9 is a maximum plasma oestradiol concentration (Cmax) following vaginal administration of 1.2 mg, 2.5 mg or 7.2 mg oestradiol 17β with γ-cyclodextrin to oestradiol 17β molar ratio of 0.5 (closed diamond), 1 (closed square) or 1.5 (closed triangle). Error bars are standard error means (n=4).
**Figure 10** is a plasma oestradiol concentration at time=0 and time=24 hours post vaginal administration of various doses of oestradiol 17β (1.2, 2.5 or 7.2 mg) with various rations of γ-cyclodextrin (0.5, 1, 1.5 molar ration of γ-cyclodextrin to oestradiol 17β). Error bars are standard error means (n=4). * Denotes a significant difference between the plasma oestradiol concentration at time=0 and time=24 hours (p<0.050).

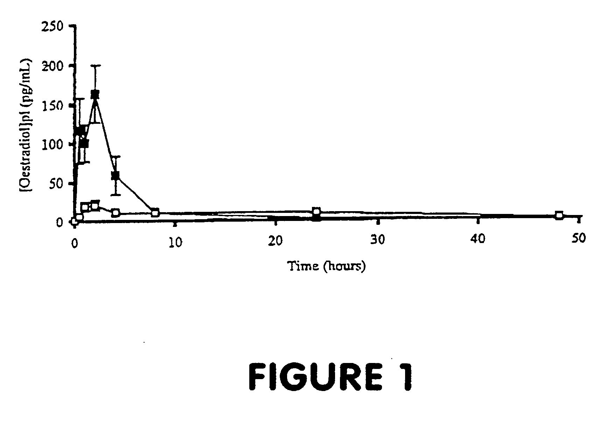

The use of oestradiol 17β and not the synthetic analogue oestradiol benzoate has not been firmly established due to the poor results with vaginally administered oestradiol benzoate and a perception that the shorter acting oestradiol 17β would not be as effications as the longer acting oestradiol benzoate. When oestradiol 17β in a dose equivalent to 1 mg oestradiol benzoate, i.e. 0.72 mg, is administered by i.m. injection in cattle the plasma oestradiol concentration rapidly rises to a maximum of approximately 100 pg/ml, followed by a rapid decline to pre injection levels by 24 hours following injection. See Figure 1. Because of this rapid decline in plasma oestradiol levels an oestradiol 17β dose of 5 mg is commonly used to ensure adequate plasma oestradiol concentrations to achieve the same effect as 1 mg of oestradiol benzoate.

We have found that unlike oestradiol benzoate vaginally administration oestradiol 17β is well absorbed, with a dose of 7.2 mg achieving a peak plasma concentration of between 10 and 20 pg/ml within four hours following administration, and the plasma oestradiol levels are elevated for at least 24 hours when oestradiol 17β is vaginally administered compared to the more rapidly cleared i.m. injection of oestradiol 17 .

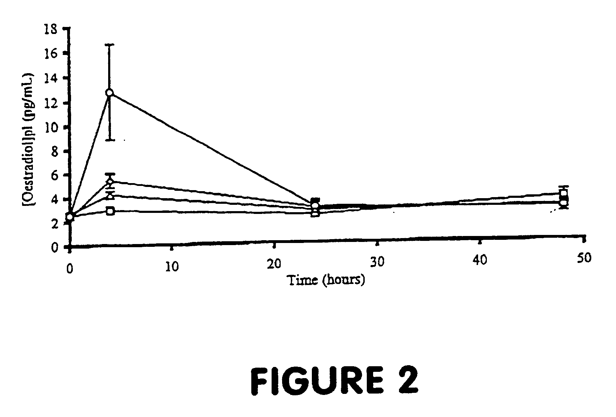

We have found that the cyclodextrins improve the vaginal absorption of oestradiol benzoate. We have found that β or γ-cyclodextrin approximately double the plasma oestradiol concentration when vaginally administered with oestradiol benzoate (10 mg) compared with oestradiol benzoate administered without cyclodextrin. See Figure 2.

Furthermore we have found that the cyclodextrin hydroxypropyl β-cyclodextrin elevates plasma oestradiol concentrations approximately 6 fold following vaginal administration with oestradiol benzoate (10 mg) compared with oestradiol benzoate administered without cyclodextrin.

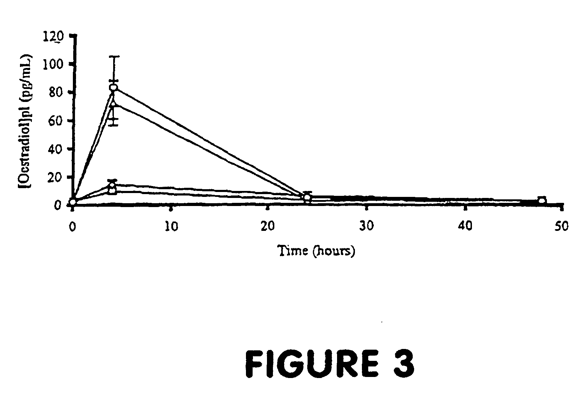

We have also found that the cyclodextrins have indeed improved the vaginal absorption of oestradiol 17β. We have found that -cyclodextrin will approximately double the plasma oestradiol concentration when vaginally administered with oestradiol 17β (7.2 mg) compared with oestradiol 17β administered without cyclodextrin. See Figure 3. Furthermore, we have found that the cyclodextrin hydroxypropyl β-cyclodextrin or γ-cyclodextrin elevates plasma oestradiol concentrations approximately 7 to 8 fold following vaginal administration with oestradiol (7.2 mg) compared with oestradiol 17β administered without cyclodextrin.

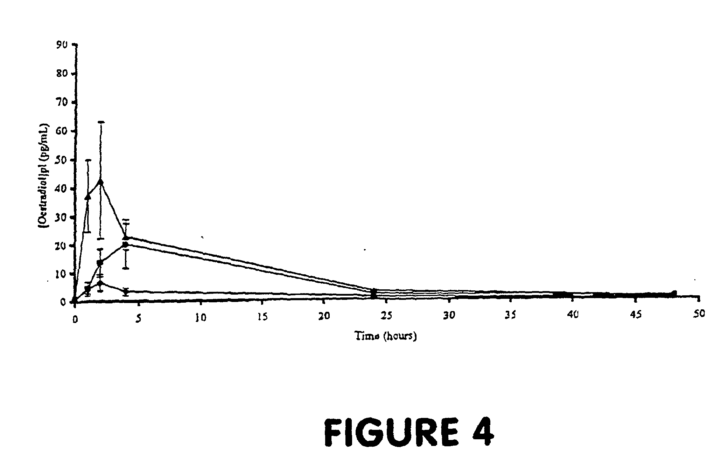

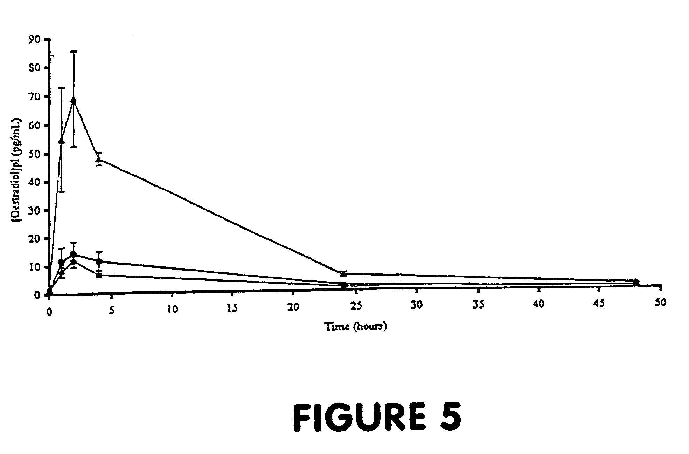

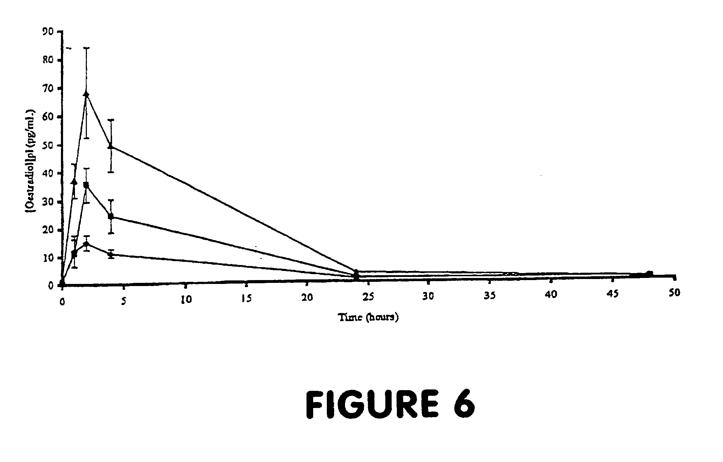

We have found that the molar ratio of γ-cyclodextrin to oestradiol 17β influences the vaginal absorption of oestradiol 17β. Increasing the ratio of γ-cyclodextrin to oestradiol 17β from 0.5:1 to 1:1 has been found to increase the plasma oestradiol concentration. See figures 4,5 and 6.

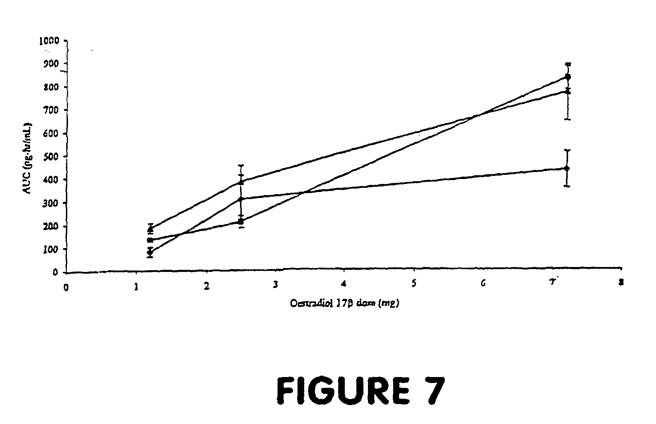

Further more the effect of the ratio of γ-cyclodextrin to oestradiol 17β upon the vaginal absorption of oestradiol 17β is more pronounced at higher doses (>2.5 mg). See figure 7.

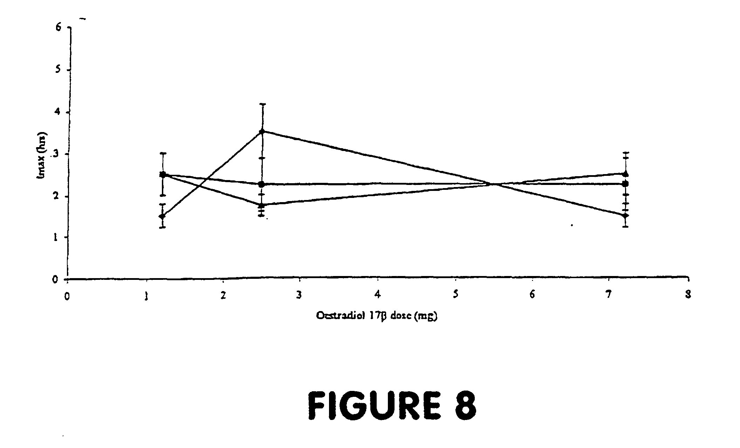

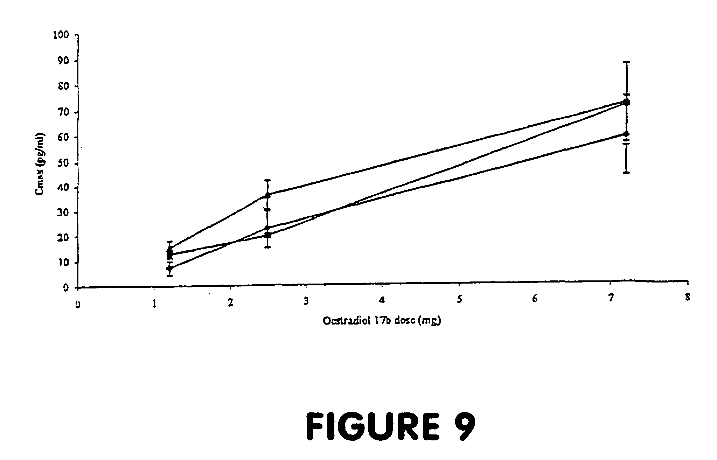

We have found that vaginal administration of various amounts of oestradiol 17β (1.2, 2.5 and 7.2 mg) with various molar ratios of γ-cyclodextrin (0.5:1, 1:1 and 3:2) to oestradiol 17β has no significant effect upon the time to maximum plasma concentration (tmax) or the maximum plasma concentration (Cmax) of oestradiol. See figures 8 and 9.

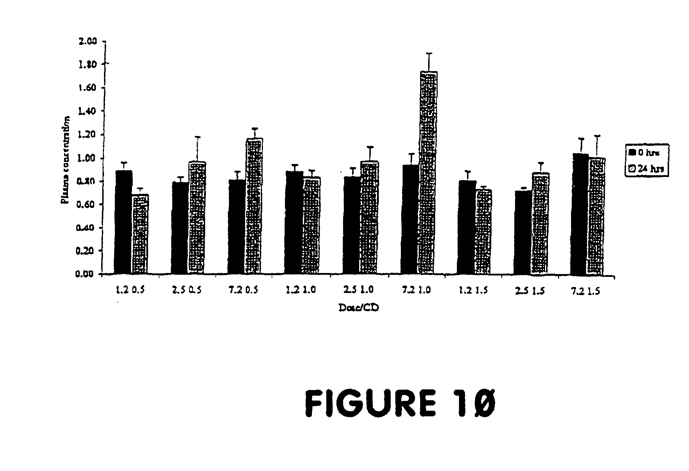

We have found that the vaginal administration of a dose of oestradiol 17β greater than 2.5 mg with an amount of γ-cyclodextrin less than or equal to a molar ratio of 1:1 (γ-cyclodextrin to oestradiol 17β) results in plasma oestradiol concentrations 24 hours post administration significantly greater than those observed prior to administration. See figure 10.

We have found vaginal administration of an oestradiol 17β dose of 5 mg and γ-cyclodextrin in a molar ratio of 0.5:1 (γ-cyclodextrin to oestradiol 17β) influences follicular dynamics in a similar manner to those observed following i.m. injection of 2 mg of oestradiol benzoate. See Table 1

Table 1 tabulates a follicular dynamics and plasma oestradiol pharmacokinetics following vaginal administration of oestradiol 17β 2.5 mg or 5.0 mg and intramuscular administration of oestradiol benzoate 2 mg.

**Table 1**

| | 2.5 mg E-17 | 5.0 E-17 | 2 mg ODB |
|---|---|---|---|
| Follicular dynamics | | | |
| n | 8 | 8 | 7 |
| Day of oestrus | 22.0 ± 0.0 (n=7) | 22.0 ± 0.0 (n=8) | 22.6 ± 0.2 (n=7) |
| In oestrus by 48 h (n) | 6 | 7 | 2 |
| | | | |
| Follicle wave turnover (i.e. DF3) | 3 | 6 | 6 |
| No follicle wave turnover (i.e. no DF3) | 5 | 2 | 1 |
| | | | |
| Day DF2 emerged | 9.9 ± 0.4 ^{a} | 10.0 ± 0.6 ^{a} | 9.6 ± 0.6 ^{a} |
| Diameter DF2 on Day 13 (mm) | 9.9 ± 0.5 ^{a} | 9.3 ± 0.8 ^{a} | 9.6 ± 1.0 ^{a} |
| Growth of DF2 from Day 13-17 (mm) | 2.4 ± 0.6 ^{a} * | 0.9 ± 0.8 ^{ab} | -0.6 ± 0.7 ^{b} |
| DF2 ovulated (n) | 5 | 2 | 0 |
| Age of ovulatory DF2 (d) | 12.0 ± 0.6 | 11.5 ± 0.5 | - |
| Diameter of ovulatory DF2 (mm) | 15.6 ± 0.7 | 17.0 ± 1.0 | - |
| | | | |
| Day DF3 emerged | 18.3 ± 0.5 | 15.7 ± 0.6 | 17.0 ± 0.4 |
| Interval, treatment to emergence of DF3 (d) | 5.3 ± 0.7 (4-6) | 2.7 ± 0.6 (1-5) | 4.0 ± 0.4 (3-5) |
| DF3 ovulated (n) | 1 | 6 | 5 |
| Age of ovulatory DF3 (d) | 6 | 7.0 ± 0.5 | 6.0 ± 0.5 |
| Diameter of ovulatory DF3 (mm) | 12 | 13.7 ±0.7 | 14.2 ± 0.4 |
| | | | |
| **Pharmacokinetics** | | | |
| | | | |
| Cmax (pg/ml) | 8.8 ± 4.3 ^{a} | 7.1 ± 4.1 ^{a} | |
| Tmax (hours) | 4.9 ± 7.8 ^{a} | 5.9 ± 7 4 ^{a} | |
| AUC (pg-hr/ml) | 58.3 ± 25.8 ^{a} | 97.8 ± 49.0 ^{a} | |
| [oestradiol]pl (pg/ml) at time=0 hours | 0.17 ± 0.06 ^{a1} | 0.89 ± 0.13 ^{b1} | |
| [oestradiol]pl (pg/ml) at time=24 hours | 0.15 ± 0.04 ^{a1} | 1.26 ± 0.35 ^{b2} | |

| | | | |
|---|---|---|---|
| ^{ab} denotes difference between groups within rows to 95% level of confidence (p<0.05) | | | |
| ¹² denotes difference between groups within columns to 95% level of confidence (p<0.05) | | | |

## Claims

1. An intra-vaginal composition comprising a solids admixture of an active principle selected from the group consisting of (i) oestradiol benzoate or prodrugs therof, (ii) oestradiol 17β or prodrugs thereof, and γ-cyclodextrin, hydroxypropyl β-cyclodextrin, or both.

2. The composition of claim 1 wherein the composition is in the form of a tablet or as part of a capsule.

3. The composition of claim 1, wherein said active principal elevates the blood serum levels of oestradiol in said mammal.

4. The composition of claim 1 wherein the composition comprises oestradiol 17β in an amount from 1.2 to 7.2 mg.

5. The composition of claim 1 wherein the composition comprises oestradiol 17β in an amount of about 0.72 mg.

6. The composition of claim 1, wherein the composition comprises oestradiol 17β and from 0.5 to 1.5 moles of γ-cyclodextrin per mole of oestradiol 17β.

7. A composition of claim 1 wherein the molar ratio of oestradiol 17β to hydroxypropyl β-cyclodextrin in the composition is 1:1.

8. A composition of claim 1 wherein the molar ratio of γ-cyclodextrin is from 0.5 to 1.5 moles per mole of oestradiol 17β.

9. A composition as defined in any one of claims 1 to 8 for use as a medicament.

10. Use of a composition as defined in any one of claims 1 to 8 for the manufacture of a medicament for the expression of oestrus in a mammal.

11. A use as claimed in claim 10, wherein the medicament is suitable for synchronisation of oestrus in mammals.

12. Use of a composition according to claim 10 in the manufacture of a vaginal medicament to achieve a plasma oestradiol concentration greater than 5 pg/ml in the mammal 2 hours following insertion and less than 5 pg/ml 24 hours following insertion of the expression of oestrus in a mammal.

## Patentansprüche

1. Intravaginale Zusammensetzung umfassend eine Feststoffmischung eines aktiven Grundbestandteils, ausgewählt aus der Gruppe von (i) Östradiolbenzoat oder dessen Wirkstoffvoriäufem, (ii) Östradiol 17β oder dessen Wirkstoffvorläufern und γ-Cyclodextrin oder/und Hydroxypropyl-β-cyclodextrin.

2. Zusammensetzung nach Anspruch 1, worin die Zusammensetzung in Form einer Tablette oder als Teil einer Kapsel vorliegt.

3. Zusammensetzung nach Anspruch 1, worin der aktive Grundbestandteil die Blutserumwerte an Östradiol in dem Säuger erhöht.

4. Zusammensetzung nach Anspruch 1, worin die Zusammensetzung Östradiol 17β in einer Menge von 1,2 bis 7,2 mg umfasst.

5. Zusammensetzung nach Anspruch 1, worin die Zusammensetzung Östradiol 17β in einer Menge von ungefähr 0,72 mg umfasst.

6. Zusammensetzung nach Anspruch 1, worin die Zusammensetzung Östradiol 17β und 0,5 bis 1,5 mol γ-Cyclodextrin pro mol Östradiol 17β umfasst.

7. Zusammensetzung nach Anspruch 1, worin das molare Verhältnis von Östradiol 17β zu Hydroxypropyl-β-cyclodextrin in der Zusammensetzung 1:1 beträgt.

8. Zusammensetzung nach Anspruch 1, worin das molare Verhältnis von γ-Cyclodextrin 0,5 bis 1,5 mol pro mol Östradiol 17β beträgt.

9. Zusammensetzung wie in einem der Ansprüche 1 bis 8 definiert zur Verwendung als Medikament.

10. Verwendung einer Zusammensetzung wie in einem der Ansprüche 1 bis 8 definiert zur Herstellung eines Medikaments für die Östrusexpression in einem Säuger.

11. Verwendung nach Anspruch 10, worin das Medikament zur Östrus-Synchronisierung in Säugern geeignet ist.

12. Verwendung einer Zusammensetzung nach Anspruch 10 zur Herstellung eines Vaginalmedikaments, um eine Plasmaöstradiolkonzentration zu erzielen, die in dem Säuger 2 Stunden nach Insertion größer als 5 pg/ml ist und 24 Stunden nach Insertion der Östrusexpression in einem Säuger niedriger als 5 pg/ml ist.

## Revendications

1. Composition intravaginale comprenant un mélange de produits solides à titre de principe actif choisis parmi le groupe constitué par : (i) du benzoate d'oestradiol ou ses précurseurs ; (ii) de l'oestradiol 17β ou ses précurseurs, et de la γ-cyclodextrine, de l'hydroxypropyl β-cyclodextrine, ou les deux.

2. Composition selon la revendication 1, dans laquelle la composition se présente sous la forme d'un comprimé ou sous la forme d'une partie d'une capsule.

3. Composition selon la revendication 1, dans laquelle ledit principe actif élève les taux d'oestradiol dans le sérum sanguin dudit mammifère.

4. Composition selon la revendication 1, dans laquelle la composition comprend de l'oestradiol 17β en une quantité de 1,2 à 7,2 mg.

5. Composition selon la revendication 1, dans laquelle la composition comprend de l'oestradiol 17β en une quantité d'environ 0,72 mg.

6. Composition selon la revendication 1, dans laquelle la composition comprend de l'oestradiol 17β et de 0,5 à 1,5 mole de γ-cyclodextrine par mole d'oestradiol 17β.

7. Composition selon la revendication 1, dans laquelle le rapport molaire de l'oestradiol 17β à l'hydroxypropyl β-cyclodextrine dans la composition s'élève à 1 : 1.

8. Composition selon la revendication 1, dans laquelle le rapport molaire de la γ-cyclodextrine s'élève de 0,5 à 1,5 mole par mole d'oestradiol 17β.

9. Composition telle que définie dans l'une quelconque des revendications 1 à 8, à utiliser comme médicament.

10. Utilisation d'une composition telle que définie dans l'une quelconque des revendications 1 à 8, pour la préparation d'un médicament pour l'expression de l'oestrus dans un mammifère.

11. Utilisation selon la revendication 10, dans laquelle le médicament est approprié pour la synchronisation de l'oestrus dans des mammifères.

12. Utilisation d'une composition selon la revendication 10 dans la préparation d'un médicament vaginal pour obtenir une concentration plasmatique d'oestradiol supérieure à 5 pg/ml dans le mammifère 2 heures après l'insertion et inférieure à 5 pg/ml 24 heures après l'insertion pour l'expression de l'oestrus dans un mammifère.
